# EUROPEAN PATENT APPLICATION

(11) **EP 2 036 904 A1**
(43) Date of publication of application: **18.03.2009**
(21) Application number: 07114004.0
(22) Date of filing: 08.08.2007
(51) Int. Cl.: C07D 405/14

(54) **A process for the preparation of olmesartan medoxomil**

(71) Applicant: LEK Pharmaceuticals d.d., 1526 Ljubljana (SI)
(72) Inventor: The designation of the inventor has not yet been filed
(74) Representative: Kröger, Bernd

(57) **Abstract**

The present invention relates to a process for the preparation and purification of trityl olmesartan medoxomil and olmesartan medoxomil.

## Description

### Field of the invention

The present invention is in the field of organic synthesis and relates to a process for the preparation and purification of trityl olmesartan medoxomil and olmesartan medoxomil.

### Background of the Invention

Olmesartan medoxomil is the name commonly given to (5-methyl-2-oxo-1,3-dioxol-4-yl)methyl 1-((2'-(1*H*-tetrazol-5-yl)biphenyl-4-yl)methyl)-4-(2-hydroxypropan-2-yl)-2-propyl-1*H-*imidazole-5-carboxylate, shown as (1) below. This chemical is known as an antagonist of angiotensin-II receptors and acts as an antihypertensive agent.

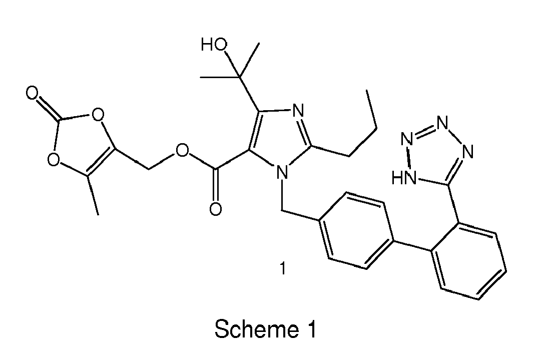

According to an article in J. Med. Chem, 1996, 39, 323-338 titled Nonpeptide Angiotensin II Receptor Antagonists, by Yanagisawa et el, olmesartan medoxomil is prepared as shown in the Scheme 2. In this process chemical intermediates are isolated in each step.

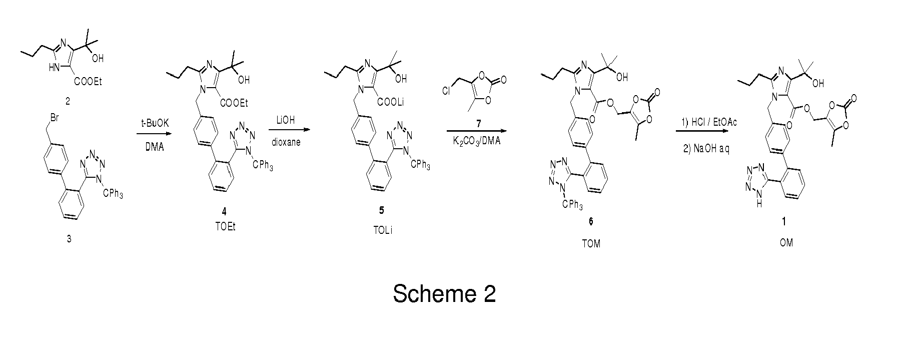

In the patent application W02007/017135 two improved processes for the synthesis of olmesartan medoxomil are disclosed.

The first process includes the step of alkylating ethyl 4-(1-hydroxy-1-methylethyl)-2-propylimidazole-5-carboxylate (2) with 4-[2-trityltetrazol-5-yl)phenyl]benzylbromide (3) and isolating the resulting product as shown below in scheme 3.

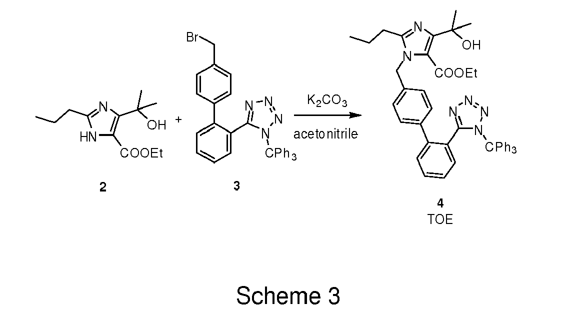

This step is followed in W02007/17135 by the steps of hydrolysis of the ethyl ester (4), the esterification with 4-substituted methyl-5-methyl-1,3-dioxolene-2-one derivative (7) and the subsequent deprotection of the trityl protection group in a one-pot process, i.e. without any isolation during the process, as shown below in scheme 4. However, the presented one-pot process includes partial purification of the intermediate trityl olmesartan medoxomil (6) by extraction and exchange of the solvent before the last reaction step.

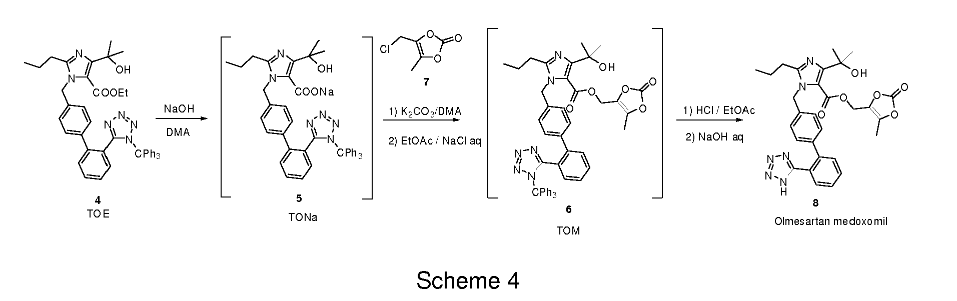

The second method provides the same alkylation reaction step with isolation of the alkylation product and an alternative synthetic approach in a one-pot process. Thus, the one-pot process comprises the hydrolysis of the ethyl ester (4), the esterification with 4-substituted methyl-5-methyl-1,3-dioxolene-2-one derivative (7) and the subsequent cycloaddition reaction of the cyano moiety forming the tetrazole group. But no experimental support is given for this approach.

It would be desirable to improve the efficiency of known processes for making olmesartan medoxomil, particularly olmesartan medoxomil of high purity.

### Summary of the Invention

According to a first aspect, the present invention provides a process for making trityl olmesartan medoxomil, the process comprising the steps:
a) alkylating ethyl 4-(1-hydroxy-1-methylethyl)-2-propylimidazole-5-carboxylate with 4-[2-(trityltetrazol-5-yl)phenyl]benzyl bromide in an organic solvent, in the presence of a base, to produce trityl olmesartan ethyl ester;
b) hydrolysing the trityl olmesartan ethyl ester in an organic solvent, in the presence of a base, to form trityl olmesartan salt; and
c) esterifying the trityl olmesartan salt with 4-chloromethyl-5-methyl-1,3-dioxolene-2-one in an organic solvent, in the presence of a base, to form trityl olmesartan medoxomil;
wherein steps a) to c) are performed without isolation of the intermediate products.

Hence, the present invention involves a "one-pot process" in which intermediates are not isolated and preferably no material is removed or exchanged In preferred embodiments, the same type solvent and base is used in steps a) to c). Hence, the process of the invention allows for improvements in efficiency to be obtained as it is possible to use less solvent and less base as it does not need to be changed during the process. The use of a one-pot solution for manufacturing trityl olmesartan medoxomil is simple and cost effective. The resultant trityl olmesartan medoxomil is a crucial intermediate in the synthesis of olmesartan medoxomil.

### Detailed description of the invention

A one-pot process for the preparation of trityl olmesartan medoxomil according to the preferred embodiments of the invention is shown below in scheme 5.

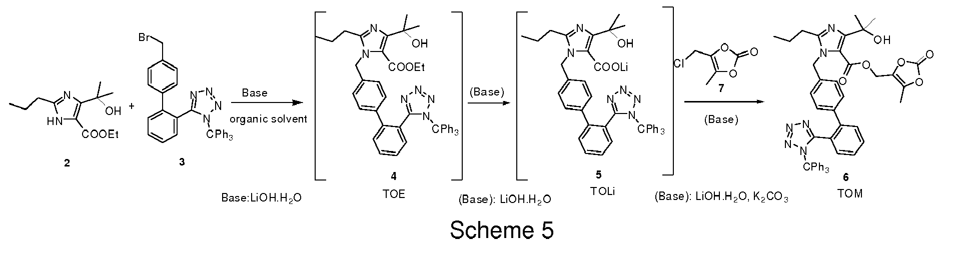

In the embodiment shown in scheme 5, lithium hydroxide hydrate is used as the base in each step, as is preferred in the invention. The use of this soft base in all of the reaction steps minimises the formation of impurities. The whole amount of base necessary can be added at the beginning of the reaction, or can be added stepwise during the one-pot process.

Hence, the first step of the invention, step a), is alkylating ethyl 4-(1-hydroxy-1-methylethyl)-2-propylimidazole-5-carboxylate (2) with 4-[2-(trityltetrazol-5-yl)phenyl]benzyl bromide (3) in an organic solvent, in the presence of a base, to produce trityl olmesartan ethyl ester (4). The reaction is started by dissolving (2) and (3) in an appropriate organic solvent and adding a base. An appropriate organic solvent is selected from a group of polar aprotic solvents such as *N,N*-dimethylformamide, *N,N*-dimethylacetamide, 1-methyl-2-pyrrolidone, dimethylsulfoxide, acetonitrile and mixtures thereof, preferably *N,N-*dimethylacetamide is used. A base can be selected from a group of alkali metal hydroxides such as lithium hydroxide, sodium hydroxide, potassium hydroxide; a group of metal carbonates such as sodium carbonate, potassium carbonate, caesium carbonate; a group of metal alkoxides or a group of organic amines, preferably alkali metal hydroxides are used, most preferable is lithium hydroxide. At the beginning of the reaction step either 1 equivalent of the base can be added and the following amount can be added step-wise during the one-pot process or the whole amount of the base can be added at the beginning of the alkylating reaction step. Preferably, 1 equivalent of the base is added at the beginning of the alkylating reaction step and the rest is added stepwise during the one-pot process. The whole amount of the added base is ranging between 2.5 to 10 equivalents, preferably between 3 and 5 equivalents. The alkylating reaction step is done by stirring the reaction mixture for 0.5 to 24 hours, preferably for 1 to 4 hours, at the temperature between 20 and 90 °C, preferably between 30 and 60 °C. After alkylating reaction step is completed the resulting product trityl olmesartan ethyl ester (4) is not isolated from the reaction mixture.

The next step, b), involves hydrolysing the trityl olmesartan ethyl ester (4) in an organic solvent, in the presence of a base, to form trityl olmesartan salt (5). This reaction step is done by simply adding the next portion of a base to the reaction mixture obtained by completion of the previous alkylation step. A base is selected from a group of alkali metal hydroxides such as lithium hydroxide, sodium hydroxide, potassium hydroxide, preferably lithium hydroxide is used. The amount of the added base is ranging between 1 and 5 equivalents, preferably 1 to 3 equivalents of the base are added. The reaction mixture is stirred for 5 to 120 hours, preferably between 24 and 72 hours at the temperature ranging from 20 to 70 °C, preferably at the temperature between 40 and 60 °C. After hydrolysing reaction step is completed the resulting product trityl olmesartan salt (5) is not isolated from the reaction mixture.

The final step in the one-pot process is step c), esterifying the trityl olmesartan salt (5) with 4-chloromethyl-5-methyl-1,3-dioxolene-2-one (7) in an organic solvent, in the presence of a base, to form trityl olmesartan medoxomil (6). This is done by optionally adding the next portion of a base to the reaction mixture obtained by completion of the previous hydrolysing step. A base can be selected from a group of alkali metal hydroxides such as lithium hydroxide, sodium hydroxide, potassium hydroxide or a group of metal carbonates such as sodium carbonate, potassium carbonate, ceasium carbonate, preferably lithium hydroxide or potassium carbonate in an amount of 0.5 to 1.5 equivalent are added to the reaction mixture. Addition of the base is followed by addition of 4-chloromethyl-5-methyl-1,3-dioxolene-2-one (7). Preferably, reagent (7) is dissolved in an organic solvent and the solution is added drop-wise to the reaction mixture, most preferably the same solvent as in reaction mixture is used for preparation of solution of (7). (7) is added in an amount of 1 to 2.5 equivalents, preferably in an amount between 1.2 to 2 equivalents. The reaction is performed by stirring the reaction mixture for 2 to 24 hours, preferably 3 to 8 hours at the temperature ranging between 20 and 70 °C, preferably at the temperature between 40 and 60 °C. After hydrolysing reaction step is completed the resulting product trityl olmesartan medoxomil (6) is isolated from the reaction mixture. The mixture is poured into large amount of water or water acetone mixture (V / V 95 : 5) and the product is precipitated from the medium. Intermediate (6) is collected by filtration and optionally purified by recrystallisation to obtain final intermediate for preparation of olmesartan medoxomil of high purity.

By "one-pot process" we mean that the relevant steps are performed in sequence without isolating the product of each step, furthermore the process of the invention is additionally simplified by omitting removal or exchange of any other component of the reaction mixture.

Using a one-pot process is a simple and cost effective method of organic synthesis but is only commercially valuable where the level of impurities can be minimised to give a reasonable yield. In the present case, the inventors have found that use of a one-pot process gives good results, particularly with the use of lithium hydroxide hydrate as a base. Such A procedure does not allow unacceptable levels of unreacted intermediates and side products to accumulate and because the one pot reaction does not include the last chemical step of preparation of the active pharmaceutical ingredient, further purification is conveniently carried out. The impurities are efficiently removed by simple recrystallisation of (6) and/or during the further step of reaction of deprotection of (6), during the isolation and purification of final product (1) in order to reach an active pharmaceutical ingredient of high chemical purity substantially free of unreacted intermediates and side products such as olmesartan ethyl ester (8) (Scheme 6).

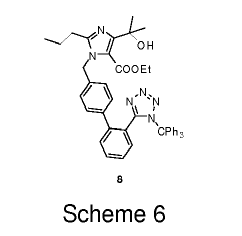

Thus, the prepared trityl olmesartan medoxomil can be further transformed into olmesartan medoxomil by any known or invented process for cleavage of trityl protection group. Scheme 7 below shows a synthetic method for deprotection of trityl olmesartan medoxomil, which may be applied in the invention.

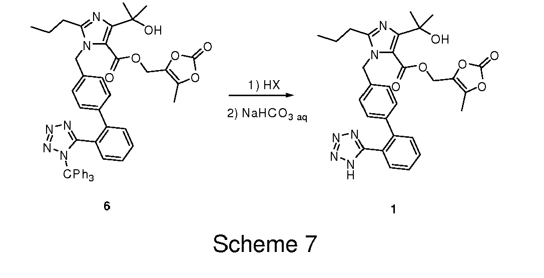

In a preferred embodiment of the invention, the process additionally comprises the steps of: d) forming a solution containing the trityl olmesartan medoxomil (6) and hydrohalic acid; e) forming olmesartan medoxomil hydrohalide salt in solid form and isolating the olmesartan medoxomil hydrohalide salt; and d) converting the olmesartan medoxomil hydrohalide salt to olmesartan medoxomil (1). This method of forming olmesartan medoxomil is particularly advantageous as it leads to high purity.

In this embodiment of the invention, there are essentially two possible routes. In the first, (6) is deprotected in the solution containing hydrohalic acid comprises a mixture of one or more water miscible organic solvents and water. The water miscible organic solvent is preferably selected from the group consisting of a C₁ to C₆ alcohol, a C₁ to C₆ ketone, a C₁ to C₆ nitrile, a C₁ to C₆ amide, a C₁ to C₆ ether, dimethyl sulfoxide, or mixtures thereof, wherein the water miscible organic solvent preferably comprises acetone, acetonitrile, ethanol, t-butanol, or 1,4-dioxane and most preferably comprises acetone.

Hydrohalic acids are preferably used as water solutions in concentrations above 10 w/w %, most preferably commercial concentrated acids like 48 % or 62 % hydrobromic acid or 35-38 % hydrochloric acid. 48 % hydrobromic acid is the most preferable. The organic solvent to water ratio is preferably between 10:1 and 1:4 by volume, more preferably between 4:1 and 1:1 by volume.

In this case, the deprotection, preferably detritylation reaction is carried out at temperatures from 20 °C to reflux preferably from 25 to 30 °C. Prior to separating the precipitated triphenylmethanol, water is added to the mixture to change the organic solvent to water ratio to about 1:3 to about 1:5, preferably to about 1:4. Triphenylmethanol is subsequently separated from the solution by any means known in the art, such as centrifugation or filtration.

The filtrate is concentrated to completely or partially remove organic solvents and the resulting aqueous suspension is stirred at temperatures ranging from 0 °C to room temperature to achieve maximum yield, then filtered to collect olmesartan medoxomil hydrohalide salt.

In the second possible route of this embodiment of the invention, (6) is deprotected in tetrahydrofuran. The detritylation reaction is carried out at temperatures from 20 °C to reflux preferably from 25 to 30 °C, and isolation is performed by cooling to -10 to 5 °C preferably to around 0 °C. In this case triphenylmethanol remains dissolved, while olmesartan medoxomil hydrohalide salt is precipitated by said cooling or by adding antisolvent selected from aromatic or aliphatic hydrocarbons and acyclic ethers, particularly preferably acyclic ethers, most preferably diisopropylether.

Olmesartan medoxomil hydrohalide salt can optionally be recrystallised, preferably from tetrahydrofuran.

In order to convert the olmesartan medoxomil hydrohalide salt to olmesartan medoxomil, the olmesartan medoxomil hydrohalide salt is dissolved in the mixture of at least one water miscible solvent and water. Suitable water miscible organic solvents include, but are not limited to, acetone, acetonitrile, lower alcohols, tetrahydrofuran, 1,4-dioxane, dimethyl sulfoxide, *N,N*-dimethylformamide, *N,N*-dimethylacetamide. Alcohols, acetone and acetonitrile are preferred; acetone is the most preferred. The organic solvent to water ratio is preferably between 2:1 and 1:3 by volume, more preferably about 1:2 by volume.

To this solution is added aqueous solution of inorganic base selected from alkali and alkaline earth carbonates, hydrogen carbonates, hydroxides, alkoxides, preferably hydrogen carbonates, more preferably NaHCO₃. The amount of base used should be such to raise the pH to about 5 to 8, more preferably 5.5 to 6.5. The temperature of the mixture should be maintained at about 0 to about 30 °C, more preferably at about 20 to about 25 °C, until olmesartan medoxomil is precipitated, and then at about 0 to about 5 °C to achieve maximum yield.

The precipitated olmesartan medoxomil is collected using any method known in the art, such as centrifugation or filtration.

Optionally olmesartan medoxomil may be recrystalized from a suitable solvent such as acetone, acetonitrile, methanol, ethanol, propanol, 2-propanol, methyl acetate, ethyl acetate, isopropyl acetate and mixtures thereof or mixtures thereof with water; preferably acetone and acetonitrile, more preferably acetonitrile.

### Examples

The following examples that illustrate the invention:

### Example 1: - one-pot synthesis of trityl olmesartan medoxomil (6)

4 g (16.7 mmol) of ethyl 4-(1-hydroxy-1-methylethyl)-2-propylimidazole-5-carboxylate (2), 9.28 g (16.7 mmol) of 4-[2-(trityltetrazol-5-yl)phenyl]benzylbromide (3) and 0.7 g (16.7 mmol) of lithium hydroxide hydrate are suspended in 70 mL of *N,N*-dimethylacetamide.

The reaction mixture is stirred for 3 h at 50 °C and then an additional 1.05 g (25 mmol) of lithium hydroxide hydrate is added to the reaction mixture. The reaction mixture is stirred for the next 40 hours at 50 °C, which is followed by addition of the third portion of lithium hydroxide hydrate (0.35 g, 8.33 mmol) and portion-wise addition of a solution of 3.84 g (24.6 mmol) of 4-chloromethyl-5-methyl-1,3-dioxolene-2-one (7) (94 %) in 10 mL of *N,N-*dimethylacetamide.

A further 7 hours of stirring the reaction mixture at 50 °C is needed to complete the reaction. In order to precipitate the product the reaction mixture is poured into 400 mL of water and the formed suspension is stirred overnight. The precipitate is filtered off and washed with 200 mL of water. The wet filter cake is recrystallised from acetone yielding 10.16 g (76 %) of trityl olmesartan medoxomil (6) (HPLC purity 93.1 area %).

### Example 2: - one-pot synthesis of trityl olmesartan medoxomil (6)

4 g (16.7 mmol) of ethyl 4-(1-hydroxy-1-methylethyl)-2-propylimidazole-5-carboxylate (2), 9.28 g (16.7 mmol) of 4-[2-(trityltetrazol-5-yl)phenyl] benzylbromide (3) and 1.75 g (41.6 mmol) of lithium hydroxide hydrate are suspended in 70 mL of *N,N*-dimethylacetamide.

The reaction mixture is stirred for 46 hours at 50 °C and then 2.53 g (18.32 mmol) of K₂CO₃ and portion-wise 4.0 g (23.0 mmol) of 4-chloromethyl-5-methyl-1,3-dioxolene-2-one (7) (85 %) in 10 mL of *N,N*-dimethylacetamide are added. The reaction mixture is stirred at 50 °C for the next 5 hours to complete the reaction.

The product is precipitated from the reaction mixture by pouring it into 400 mL of a mixture of water and acetone (V / V = 95 / 5). The formed suspension is stirred overnight and the precipitate is filtered off and washed with 200 mL of water. The wet filter cake is recrystallised from acetone yielding 9.95 g (75 %) of trityl olmesartan medoxomil (6) (HPLC purity 97.7 area%).

### Example 3: - one-pot synthesis of trityl olmesartan medoxomil (6)

4 g (16.7 mmol) of ethyl 4-(1-hydroxy-1-methylethyl)-2-propylimidazole-5 carboxylate (2), 9.28 g (16.7 mmol) of 4-[2-(trityltetrazol-5-yl)phenyl]benzylbromide (3) and 0.7 g (16.5 mmol) of lithium hydroxide hydrate are suspended in 70 mL of *N,N*-dimethylacetamide. The reaction mixture is stirred for 45 min and then next portion of lithium hydroxide hydrate (2.10 g, 50 mmol) is added to the reaction mixture. After stirring for 48 hours at 50 °C portion-wise 4.9 g (31.0 mmol) of 4-chloromethyl-5-methyl-1,3-dioxolene-2-one (7) (94 %) in 10 mL of *N,N*-dimethylacetamide is added. The reaction mixture is stirred further at 50 °C for 6 hours to complete the reaction. The product is precipitated from the reaction mixture by pouring it into 400 mL of a mixture of water and acetone (V / V = 95 / 5). The formed suspension is stirred overnight and the precipitate is filtered off and washed with 200 mL of water. The wet filter cake is recrystallised from acetone yielding 9.56 g (72 %) of trityl olmesartan medoxomil (6) (HPLC purity 96.3 area %).

### Preparation procedure 4: - transformation of trityl olmesartan medoxomil (6) to olmesartan medoxomil (1)

10 g (12.5 mmol) of trityl olmesartan medoxomil (6) prepared by the example 1 is suspended in 50 mL of a mixture acetone and water (V / V = 3 : 1). 4.75 mL of 48 % hydrobromic acid is added to the suspension. After stirring the reaction mixture for 2 hours 100 mL of water is added. The precipitated triphenylmethanol is filtered off. Acetone is evaporated from the filtrate. The resulting concentrate is stirred at room temperature for 0.5 hour and for additional 1 hour at 0 °C. The precipitated olmesartan medoxomil hydrobromide is filtered (1) (HPLC purity 99.80 area %).

## Claims

1. A process for making trityl olmesartan medoxomil, the process comprising the steps of:
a) alkylating ethyl 4-(1-hydroxy-1-methylethyl)-2-propylimidazole-5-carboxylate (2) with 4-[2-(trityltetrazol-5-yl)phenyl]benzyl bromide (3) in an organic solvent, in the presence of a base, to produce trityl olmesartan ethyl ester (4);
b) hydrolysing the trityl olmesartan ethyl ester (4) in an organic solvent, in the presence of a base, to form trityl olmesartan salt (5); and
c) esterifying the trityl olmesartan salt (5) with 4-chloromethyl-5-methyl-1,3-dioxolene-2-one (7) in an organic solvent, in the presence of a base, to form trityl olmesartan medoxomil (6);
wherein steps a) to c) are performed without isolation of the intermediate products.

2. A process according to claim 1, wherein the same type of organic solvent is used in each of steps a) to c), preferably wherein the organic solvent is N,N-dimethylacetamide.

3. A method according to claim 1 or 2, wherein the same type of base is used in each of steps a) to c), preferably wherein the base is lithium hydroxide hydrate.

4. A method according to any preceding claim, additionally comprising the steps of:
d) forming a solution containing the trityl olmesartan medoxomil (6) and hydrohalic acid;
e) forming olmesartan medoxomil hydrohalide salt in solid form and isolating the olmesartan medoxomil hydrohalide salt; and
f) converting the olmesartan medoxomil hydrohalide salt to olmesartan medoxomil (1).
